# EUROPÄISCHE PATENTSCHRIFT

(11) **EP 0 444 021 B1**
(45) Veröffentlichungstag und Bekanntmachung des Hinweises auf die Patenterteilung: **25.01.1995**
(21) Anmeldenummer: 88909734.1
(22) Anmeldetag: 14.11.1988
(51) Int. Cl.: A61N 1/365, A61N 1/368

(54) **HERZSCHRITTMACHER**
CARDIAC PACEMAKER
STIMULATEUR CARDIAQUE

(30) Priorität: 13.11.1987 DE 3739091
(43) Veröffentlichungstag der Anmeldung: 04.09.1991
(73) Patentinhaber: BIOTRONIK Mess- und Therapiegeräte GmbH & Co Ingenieurbüro Berlin, D-12359 Berlin (DE)
(72) Erfinder: SCHALDACH, Max, D-8520 Erlangen (DE)
(74) Vertreter: Christiansen, Henning, Dipl.-Ing.
(86) Internationale Anmeldenummer: DE8800723
(87) Internationale Veröffentlichungsnummer: WO8904192

(56) Entgegenhaltungen:
- EP-A- 0 249 680
- EP-A- 0 249 820
- GB-A- 2 026 870

## Beschreibung

Die Erfindung betrifft einen Herzschrittmacher der im Oberbegriff des Anspruchs 1 angegebenen Art.

Bei neuzeitlichen Herzschrittmachern werden eine Reihe von Eingangssignalen verarbeitet, die an getrennten Leitungen anfallen. Diese Signale müssen jeweils entsprechend dem Signalpegel in der Amplitude heraufgesetzt und gegebenenfalls gefiltert oder zwischengespeichert werden. Dazu sind bei den bekannten Schrittmachern getrennte Eingangsverstärker notwendig, welche in konventioneller Ausführung ein beträchtliches Bauvolumen erfordern, das der geforderten Verkleinerung der Außenmasse von implantierbaren Herzschrittmachern entgegensteht. Auch in integrierter Ausführung ist die durch mehrere Vorverstärker und Filter belegte Fläche auf einem Siliziumchip beträchtlich. Dazu kommt, daß die Schaltungen in Analogausführung im Vergleich zu digitalen Schaltungen recht aufwendig sind und eine Vielzahl von integrierten Bauelementen erfordern, die den zusätzlichen Aufwand für jeden Verarbeitungskanal beträchtlich werden lassen.

Aus GB-A 2 026 870 ist es zu diesem Zweck bekannt, die Eingangssignale verschiedener Signalaufnehmer einem Multiplexer zuzuführen, der - durch einen Mikroprozessor des Schrittmachers gesteuert - die Signale der einzelnen Aufnehmer zeitsequentiell einem A/D-Wandler/Verstärker zuleitet, der sie in zur Verarbeitung durch den Mikroprozessor geeignete digitale Form umwandelt.

Dieser gattungsgemäße Herzschrittmacher ist jedoch, da die Signalübertragungscharakteristik des Signalweges nicht veränderbar ist, nur für Signalaufnehmer geeignet, die in ihren Charakteristika vergleichbare Signale liefern.

Der Erfindung liegt daher die Aufgabe zugrunde, einen Herzschrittmacher anzugeben, bei dem eine die spezifischen Charakeristika unterschiedlicher Signalaufnehmer berücksichtigende Signalverarbeitung auf schaltungstechnisch rationelle Weise erfolgt.

Diese Aufgabe wird durch die im kennzeichnenden Teil des Anspruchs 1 angeführten Maßnahmen gelöst.

Die Erfindung beruht auf der Erkenntnis, daß die bei der Signalverarbeitung im Herzen auszuwertenden Signale hinsichtlich der zu berücksichtigenden zeitlichen Änderungen eine weitaus geringere Übertragungsbandbreite benötigen als übliche Verstärkerschaltungen bieten.

Durch die zeitlich überlagerte Betriebsweise können in ein und demselben Übertragungskanal sämtliche oder nahezu sämtliche für das Betriebsverhalten eines Herzschrittmachers relevanten Signale verarbeitet werden.

Besonders vorteilhaft dabei ist, daß gleichzeitig mit der sequentiellen Umschaltung zwischen Signalquellen, Übertragungsweg und Ausgangsanschlüssen auch die Übertragungseigenschaften des Übertragungswegs durch Umschaltung von Verstärkungsfaktoren und/oder Filtereigenschaften insbesondere mittels entsprechend synchron umschaltbarer Kapazitäten umschaltbar sind. Hierbei fügen sich die Umschaltungen in die sequentiellen Taktzeiten ein, so daß ein Umschaltvorgang eines Signalwegs darin besteht, daß im Verlauf der wiederkehrenden Taktsequenz unterschiedliche Signalwege periodisch eingeschaltet werden. Daneben können aber auch Signale für unterschiedliche Betriebsweisen innerhalb einer wiederkehrenden Taktsequenz übertragen werden, so daß die Signale für diese Betriebsweisen hinsichtlich der Signalverarbeitung quasi "gleichzeitig" verarbeitet werden können, wobei erst in einer nachfolgenden Stufe dasjenige Signal ausgewählt wird, welches das beste Ergebnis bietet.

Der Übertragungsweg kann gleichzeitig für Signalformungs- und Regelzwecke eingesetzt werden, wobei die Eigenschaften des Übertragungswegs auch geregelt verändert werden können, wobei eine Rückkopplung in der Weise erfolgt, daß die Eigenschaften der Leitung in Abhängigkeit von den über diese übertragenen Daten veränderbar sind, wobei in vorteilhafter Weiterbildung der Übertragungsweg selbst bzw. ein zeitsequentieller Teil desselben auch die Regelstrecke bilden kann.

Damit ergibt sich eine optimierte Verstärkerkonfiguration für implantierbare Herzschrittmacher. Die Konfiguration erfordert minimale externe passive Komponenten und eine minimale Anzahl von IC-Schaltungen, um einen Multiplex-Zweikammerbetrieb einschließlich atrialer und ventrikulärer endokardialer EKG-Überwachung und Signalaufnahme zu gewährleisten. Außerdem ist die bipolare und unipolare Betriebsweise programmierbar sowie das Austasten des Eingangs und programmierbare Empfindlichkeit (Verstärkung). Auf dem Chip ist eine mehrpolige Multiplex-Bandpaß-EKG-Filterschaltung vorgesehen, um R-Wellen und P-Wellen-Signale zu erfassen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein prinzipielles Blockschaltbild eines Ausführungsbeispiels des erfindungsgemäßen Herzschrittmachers,
Figuren 2a und 2b ein detaillierteres Blockschaltbild entsprechend demjenigen gemäß Figur 1,
Figur 3 ein Detailschaltbild des Multiplex-Übertragungswege enthaltenden Verarbeitungsteils gemäß Figur 2b,
Figuren 4 bis 6 Einzelheiten von Schaltungen des Multiplex-Verarbeitungsteils gemäß Figur 3 sowie
Figur 7 ein entsprechendes Zeitdiagramm.

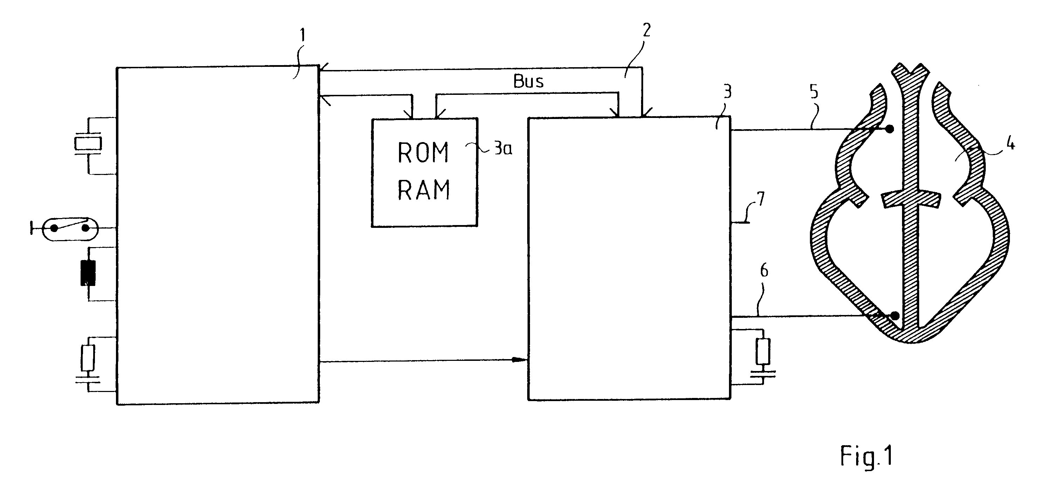

Bei dem in Figur 1 dargestellten Blockdiagramm ist eine Controller-Einheit 1 über einen Bus 2 verbunden mit einer Analog-Einheit 3. An den Bus 2 angeschlossen ist weiterhin ein Speicher 3a, bestehend aus ROM- und RAM-Speicherbereichen. An den Analogteil 3 sind die Verbindungsleitungen zum Herzen 4 angeschlossen sowie weitere Sensoren für im Körper des Patienten abzuleitende Parameter. Im dargestellten Ausführungsbeispiel sind eine Vorhof-Elektrode 5, eine Kammerelektrode 6 und ein weiterer Sensoreingang 7 dargestellt. Die Controller-Einheit 1 und die Analog-Einheit 3 sind mit weiteren Baugruppen beschaltet, welche in den nachfolgenden Blockschaltbildern näher beschrieben sind. Die in Figur 1 dargestellten Baugruppen sind zusammen mit einem Energieversorgungsteil in ein implantierbares Gehäuse hermetisch eingeschlossen und mit Anschlüssen für die externen Elektroden bzw. Sensoren versehen.

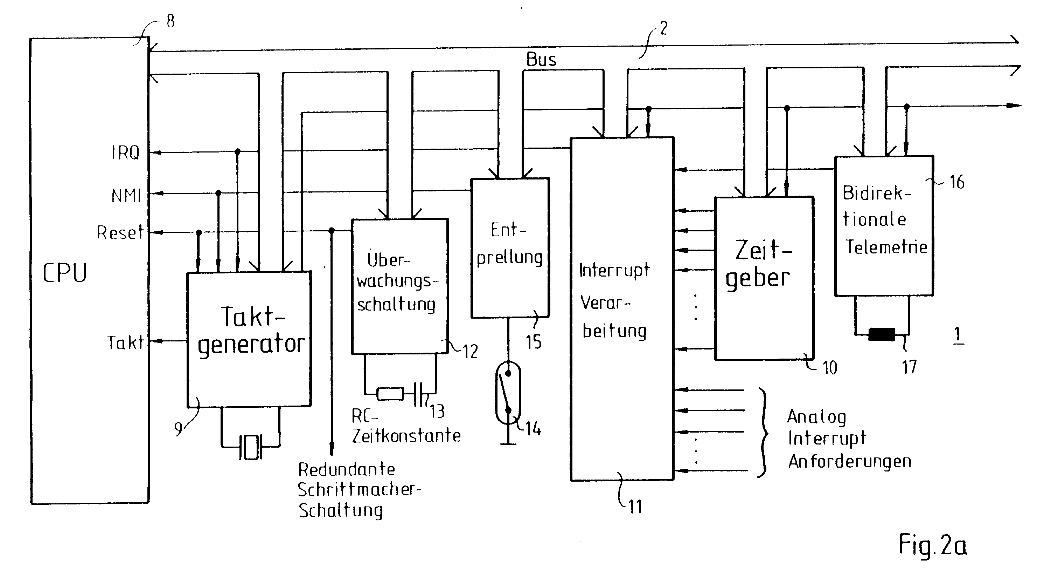

In den im Figur 2a dargestellten Blockschaltbild wird eine CPU 8 von einen quarzstabilisierten Taktgenerator 9 mit dem Systemtakt versehen. Von der CPU aus werden die digitalen Datenverarbeitungsvorgänge gesteuert, wobei die digitalen Daten zwischen den Blöcken über den Bus 2 ausgetauscht werden. Die CPU verweilt in einem Ruhezustand, wenn nicht durch externe Ereignisse oder einen Zeitgeber 9 veranlaßte Interrupts eine Behandlung durch die CPU erfordern. Sämtliche Interrupts werden zentral von einer Interrupt-Verarbeitungseinheit 11 erfaßt und über die Leitung IRQ (Interrupt-Request) der CPU mitgeteilt. Auf diese Weise werden die bekannten Schrittmacherfunktionen realisiert, wobei Stimulationsimpulse entweder in vorgegebenem Zeitabstand nach im Herzen aufgenommenen Spontan-Ereignissen oder - bei Ausfall dieser Ereignissen - im vorgegebenen Zeitabstand ausgelöst werden. Die Verknüpfung dieser Ereignisse erfolgt nach einem vorgegebenen Programm, welches in dem in Figur 1 dargestellten Speicher 3 vorgegeben ist. Dieses Programm bildet die Grundlage für die Systemsteuerung und die Übertragung von Daten zwischen den einzelnen Blöcken des Controllers. Dazu gehören weiterhin eine Überwachungsschaltung 12, welche mittels einer RC-Zeitkonstante die Stimulationsereignisse zusätzlich überwacht und eine redundante Schrittmacherschaltung im Analogteil synchronisiert, welche bei Ausfall des Digital-Systems eingreift. Die RC-Zeitkonstante 13 der Überwachungsschaltung 12 ermöglicht eine vom Systemtakt unabhängigen Zeitvergleich.

Ein konventioneller Reed-Schalter 14 ist mit einer Entprellungsschaltung 15 versehen und ermöglicht die bei Herzschrittmachern bekannten Magnetschalter-Funktionen. Weiterhin ist mit dem Bus 2 eine bidirektionale Telemetrieschaltung 16 verbunden, welche über eine Spule 17 mit einer externen Programmiereinheit kommuniziert und sowohl die "Programmierung" der Schrittmacherschaltung in der Weise ermöglicht, daß die systemeigenen Programme, und damit die Betriebsparameter des Schrittmachers, verändert werden, als auch die Übertragung von EKGs und Schrittmacherdaten aus dem Körper des Patienten heraus zuläßt.

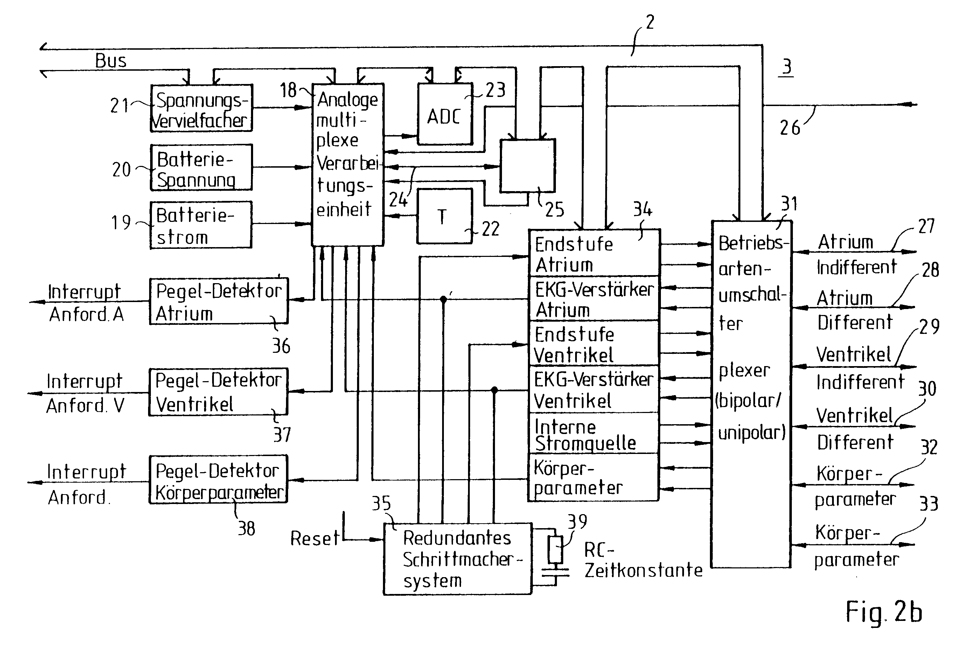

In Figur 2b ist der Analogteil des erfindungsgemäßen Schrittmachers dargestellt, wobei verschiedene Analog-Einheiten mit dem Systembus 2 zusammenarbeiten. Zentrale Baugruppe ist dabei eine analoge Multiplex-Verarbeitungseinheit 18, welche gesteuert vom Systembus verschiedene analoge Signale in einem gemeinsamen Kanal überträgt und formt. Die Taktsteuerung erfolgt dabei vom Bus 2, während die analogen Ein- und Ausgangssignale zu den nachfolgend beschriebenen analogen Einheiten gelangen.

Neben den ausschließlich für die Schrittmacherfunktionen notwendigen Signalen sind zusätzliche Baugruppen zur Überwachung von schrittmacherinternen Funktionen vorgesehen. So können der Batteriestrom 19, die Batteriespannung 20 sowie ein über den Datenbus programmierbarer Spannungsvervielfacher in ihrer Funktion durch periodische Abfrage der Ausgangsdaten überwacht werden. Als weitere innerhalb des Schrittmachers zur Verfügung stehende Größe wird von einer Temperatursensor-Einheit 22 die aktuelle Körpertemperatur des Patienten abgefragt und der Verarbeitungseinheit 18 zugeführt. Hierbei handelt es sich dann um eine Größe, welche als Maß für die körperliche Belastung auf die Schrittmacherrate Einfluß nimmt. Entsprechendes kann auch für die Daten der Energiequelle gelten, wobei es beispielsweise bekannt ist, mit Abfallen der Batteriespannung auf einzelne Leistungsmerkmale des Schrittmachers zu verzichten bzw. die Impulsabgabe ökonomischer zu gestalten. Die Ausgangssignale des analogen Multiplexers gelangen in diesem Fall zu einem Analog-Digital-Converter (ADC) 23. Am Beispiel einer Schleife 24 ist aber auch gezeigt, daß die Analog-Signale den Multiplexer-Verarbeitungsweg mehrfach durchlaufen können, wobei eine "Sample-and-hold"-Schaltung für eine Zwischenspeicherung sorgt, da diese aufeinanderfolgende Verarbeitung in unterschiedlichen "Verarbeitungsfenstern" erfolgen muß, um eine Rückkopplung zu vermeiden und um beispielsweise sicherzustellen, daß eine unterschiedliche Signalfilterung oder -zwischenspeicherung mittels Zeitkonstanten erfolgen soll.

Auf diese Weise kann beispielsweise die zeitverzögerte Änderung der Herzschlagrate bei Änderungen der Körpertemperatur mit den dabei zu berücksichtigenden Zeitkonstanten in analoger Form auf dem Verarbeitungsweg nachgebildet werden. Durch eine geringe Taktfrequenz für diesen Verarbeitungskanal verlaufen die entsprechenden Ausgleichsvorgänge in den so gebildeten Signalfilterschaltungen entsprechend langsam. Mit der analogen Multiplex-Verarbeitungseinheit steht weiterhin eine zu einem externen Anschluß des Schrittmachers führende Leitung 26 in Verbindung, welche - siehe weiter unten beschrieben - bereits in zeitlich multiplexer Überlagerung eintreffende Daten in den zeitlichen Ablauf der Verarbeitungseinheit 18 einbindet.

Die weiteren mit dem Körper in Verbindung tretenden Ausgangsleitungen, wie die beiden Atrium-Anschlüsse 27 und 28 und die beiden Ventrikel-Anschlüsse 29 und 30 lassen sich durch einen Betriebsarten-Umschalter 31, der über die Bus-Leitung 2 gesetzt werden kann, alternativ in bipolarer oder unipolarer Betriebsweise benutzen. Zusätzlich über den Betriebsarten-Umschalter 31 gelangen von Anschlüssen 32 und 33 zusätzlich im Körper des Patienten aufgenommene Signale in die entsprechende Verarbeitungseinheiten des Schrittmachers, wobei es sich in diesem Fall um Signaleingänge handelt, die mit der Elektrodenzuleitung verbunden werden. Die entsprechenden Signalaufnehmer sind dann entsprechend innerhalb der im Herzen anzubringenden Elektrode vorgesehen, wobei die Betriebsarten-Umschaltung in der Baugruppe 31 eine Anpassung an verschiedene Sensoren programmgesteuert über den Bus 2 zuläßt.

In einer weiteren Baugruppe 34, die über den Bus 2 angesprochen werden kann, sind die Eingangs- und Ausgangsstufen für die Herzstimulation und für die Signalaufnahme über die Elekrodenzuleitung vorgesehen. Es sind der Reihe nach die Endstufe (Ausgangsstufe) für die Stimulation des Atriums, der EKG-Verstärker (Sense-Eingang)-Stufe für das Atrium, die Endstufe für den Ventrikel, der EKG-Verstärker für den Ventrikel, eine interne Stromquelle für den Impedanzmessung im Herzen im Falle der Anwendung von Plethysmographie sowie eine Empfängerschaltung für Körperparameter.

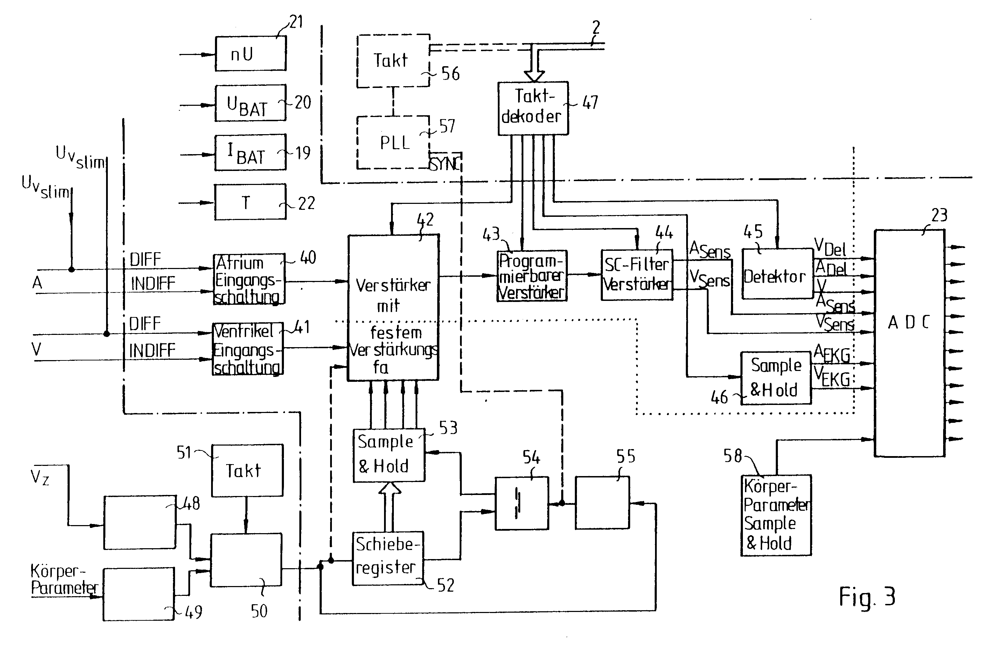

Während die Impulsausgangs-Endstufen entweder über die entsprechenden auf dem Bus 2 vorhandenen Daten angesprochen oder aber die entsprechenden Impulse direkt durch ein zusätzliches redundantes Schrittmachersystem 35 ausgelöst werden, das von der Schaltung 12 in Figur 2a synchronisiert wird, geben die Eingangsstufen ihre Signale auf die analoge Multiplex-Verarbeitungseinheit 18 ab. Hier werden sie getaktet, nacheinander in zeitlicher Folge den notwendigen Verarbeitungen unterworfen, wie es anhand der detaillierteren Darstellung in Figur 3 näher beschrieben werden soll.

Das redundante System 35 erhält weiterhin die Eingangssignale der EKG-Verstärker in Atrium und Ventrikel jeweils direkt zugeführt, um beim Ausfall des digitalen Verarbeitungsteils unabhängig stimulierend wirken zu können, wobei als zeitbestimmendes Element ein RC-Zeitkonstante 39 wirkt. Das redundante Schrittmachersystem wird während des Betriebs des digitalen Systems von der Überwachungsschaltung 12 in Figur 2a über den RESET-Eingang synchronisiert, um jederzeit die Stimulation synchron weiterführen zu können.

Die von der Multiplex-Einheit entsprechend verarbeiteten Signale gelangen von der Einheit 18 zu Pegel-Detektoren 36, 37 und 38 für die jeweiligen Eingangssignale aus Atrium, Ventrikel oder sonstigen Körperregionen, wobei die Pegeldetektoren einstufige Analog-digital-Wandler bilden. Die Ausgangssignale dieser Detektoren 36 bis 38 erzeugen Interrupt-Anforderungen bei der Interrupt-Verarbeitungseinheit 11 in Figur 2a.

In Figur 3 ist die erfindungsgemäße optimierte Verstärkerkonfiguration mit Multiplex-Verarbeitung für einen Herzschrittmacher als Blockschaltbild näher dargestellt. Der strichpunktiert umrandete Teil entspricht dem Umfang der Darstellung in Figur 2b mit Ausnahme des dort wiedergegebenen redundanten Schrittmacherteils 35.

Das Blockdiagramm gemäß Figur 3 enthält einen externen Teil mit passiver Beschaltung und einen internen IC-Schaltkreis.

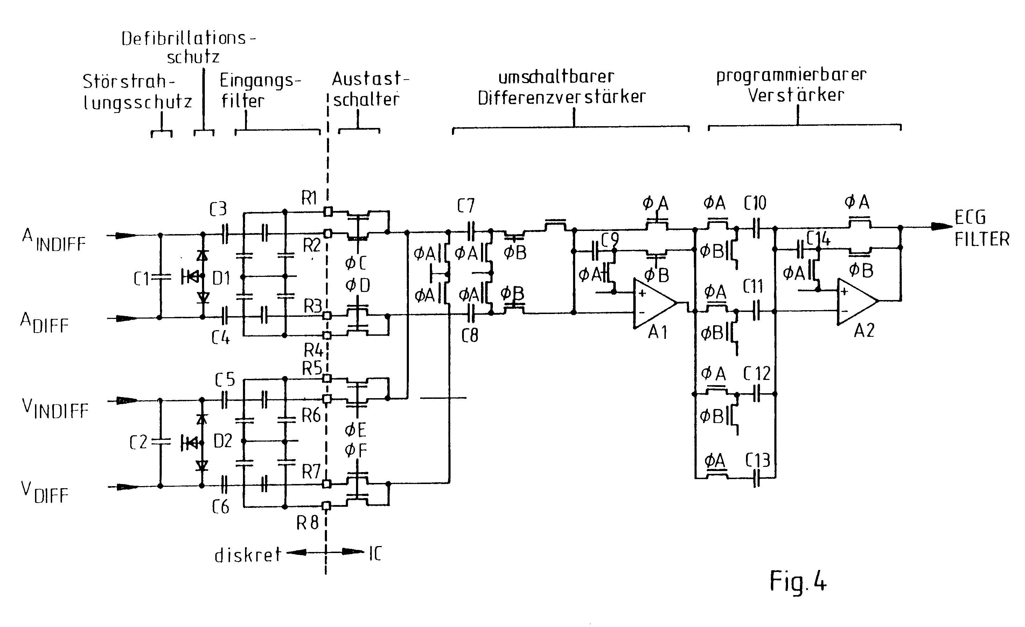

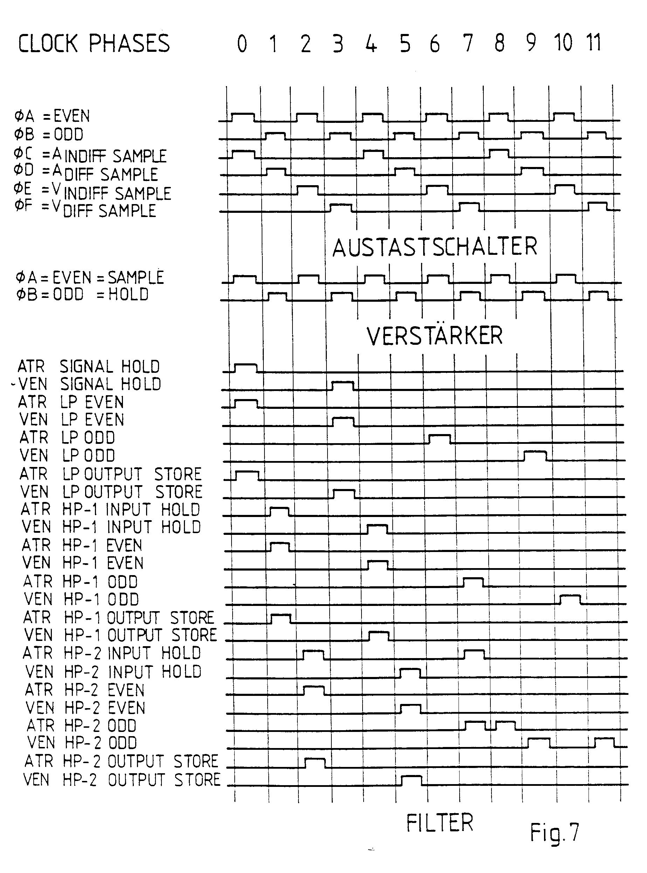

Details der Schaltungsanordnung sind anhand von beispielhaft ausgewählten Baugruppen in den Figuren 4 bis 6 wiedergegeben, während die Werte der Filterkomponenten in Tabelle 1 dargestellt sind. Die zugehörigen Zeitdiagramme finden sich in Figur 7.

Bei den in den Figuren 4 bis 7 näher dargestellten Baugruppen handelt es sich um die atriale und die ventrikuläre Eingangsschaltung 40 bzw. 41, den daran anschließenden Verstärker mit festem Verstärkungsfaktor 42, den folgenden Verstärker mit veränderlichem Verstärkungsfaktor 43, den Filter-Verstärker 44, Detektorschaltung 45 und die Sample&Hold-Schaltung 46. Die Eingänge der jeweils nachfolgend genannten Schaltung sind jeweils mit den Eingängen der vorher genannten Schaltung verbunden mit Ausnahme der Schaltungen 40 und 41, deren Ausgangssignale beide zu der Schaltung 42 gelangen, und der Schaltung 46, deren Eingang ebenfalls mit dem Ausgang der Schaltung 43 verbunden ist.

Ein Speicher/Dekoder 47 erzeugt die Steuersignale für die einzelnen Baugruppen im Multiplexbetrieb aus den auf dem Bus 2 anstehenden Signalen. Die ensprechenden Datenbytes werden in der notwendigen Folge vom digitalen Verarbeitungsteil (Controller) erzeugt. Ein entsprechendes Steuersignal auf dem Bus schaltet jeweils eine der jeweiligen Baugruppe zugeordnete Steuerleitung ein bzw. aus. Ein Zeitschema für die in den Figuren 4 bis 6 dargestellten Baugruppen ist in Figur 7 wiedergegeben. Durch das Programm des Controllers wird der Zeitgeber jeweils für den nächsten Zeitpunkt, zu dem ein entsprechendes Schaltsignal einer der Baugruppen zugeleitet werden soll, gesetzt und nach Erreichen dieses Zeitpunkts ein Interrupt ausgelöst, der den möglicherweise zwischenzeitlich in den Ruhezustand gesetzten Prozessor (CPU) aktiviert.

In der Schaltung gemäß Figur 3 ist weiterhin die Möglichkeit vorgesehen, die multiplexe Signalübertragung auch bereits auf einer Leitung zu benutzen, die eine Verbindung zumindestens einem externen Meßaufnehmer oder beispielsweise auch der Schrittmacherelektrode bildet.

Eingangsschaltungen 48 und 49, welche im Aufbau den Schaltungen 40 und 41 entsprechen, sind separat vorgesehen und ausgangsseitig mit einem Verstärker mit festem Verstärkungsfaktor 50 verbunden, der dem Verstärker 42 konstruktionsmäßig entspricht. Die Eingänge des Verstärkers 50 werden mit den Eingangsschaltungen 48 und 49 gesteuert durch einen Taktgeber 51 Zyklus verbunden, so daß am Ausgang des Verstärkers 50 die im Pegel heraufgesetzten Signale, wie sie von den Schaltungen 48 und 49 abgegeben werden ausschnittsweise zyklich erscheinen. Die Schaltungen 48 bis 51 sind in in einer Baugruppe zusammengefaßt, welche mit der übrigen Schrittmacherschaltung, die entfernt davon - bevorzugt den außerhalb eines Gehäuses - angeordnet ist und nur mit einer Datenleitung und den Leitungen für die Stromversorgung verbunden ist.

Auf diese Weise können eine Anzahl von externen Meßstellen vorgesehen werden, ohne daß innerhalb des Körpers empfindliche, jeweils eine Vielzahl von Adern enthaltende, Leitungen verlegt werden müssen. In dargestellten Ausführungsbeispiel wird das Eingangssignal für die Eingangsschaltung 48 von einer zusätzlichen im Ventrikel angebrachten Elektrode V_{z} abgeleitet, welche alle Signalerfassung unabhängig von einer Polarisierung der Stimulationselektrode ermöglicht. Das von der Hilfselektrode V_{z} abgeleitete Signal kann auch dazu dienen, die Rückladung eines Ausgangskondensatcrs der Stimulationsschaltung im Ventrikel durch sogenannte "Autoshort-Impulse", welche dem Stimulationsimpuls entgegengerichtet sind, zu überwachen, um auf diese Weise zu ermitteln, wann die Polarisierung der Stimulationselektrode soweit abgeklungen ist, daß sie wieder zur Signalaufnahme - und insbesondere zur Effektivitätserkennung - zur Verfügung steht.

Der Eingang der weiteren Schaltung 49 kann zur Ermittlung von plethysmographischen Daten dienen, welche über die elektrische Widerstandsverteilung in der Herzkammer Aussagen über die ventrikuläre Aktivität ermöglichen.

Die von der Baugruppe 50 sequentiell übertragenen Daten gelangen über die Zuleitung zu einem innerhalb des Schrittmachergehäuses befindlichen Schieberegister 52 und werden nach vollständiger Übertragung einer Datensequenz in eine Sample&hold-Schaltung 53 übertragen. Dieser Übertragungsvorgang wird ausgelöst durch einen Schalter 54, dessen Ausgangssignal die Sample&hold-Schaltung zur Aufnahme neuer Daten aktiviert. Der Schalter seinerseits wird angesteuert von einem Decoder 55, der auf ein zyklisch wiederkehrendes Signal innerhalb des Eingangssignals des Schieberegisters 52 anspricht, wobei dieses Signal das Ende einer Datensequenz anzeigt.

Im hier dargestellten Ausführungsbeispiel weist dieses Signal einen Pegel auf, der über die üblicherweise auftretenden weiteren Signalpegel hinausgeht und liegt an einem Eingang der Schaltung 50 als Signal "++" ständig an.

Infolge der zyklischen Abfrage dieser Eingänge bildet es ein Bezugssignal innerhalb der zyklisch aufeinanderfolgenden Signalproben. Die Decoderschaltung 55 stellt insoweit einen Schwellwertdetektor dar, welcher nur durch dieses in der Signalfolge erscheinende Bezugssignal zum Ansprechen gebracht wird. Die Ausgangssignale der Sample&hold-Schaltung 53 werden durch die Verstärkerschaltung 42 über deren zusätzliche Eingänge entsprechend zyklisch abgefragt, so daß die extern bereits multiplex übertragenenen Signalwerte in die Multiplex-Verarbeitung der Baugruppen 42 bis 46 übernommen werden. Bei dieser Ausführung werden die in ein Multiplexverfahren zum Schrittmacher übertragenen Daten mittels der Schaltungen 52 und 53 wieder in an verschiedenen Leitungen in analoger Form anstehende separate Signale übertragen, um mittels der Schaltung 42 erneut mittels Abtastung in Multiplex-Signale verwandelt zu werden.

Bei einer (gestrichelt dargestellten) Variante werden die durch die Baugruppe 50 gemultiplexten Daten direkt in die Multiplex-Zeitfolge der Baugruppen 42 bis 45 einbezogen. Dazu gelangen die von außerhalb gelegenen Meßwertaufnehmern ermittelten auf eine einzige Leitung gemultiplexten Daten vom Ausgang der Baugruppe 50 über die gestrichelt dargestellte Leitung direkt zu einem Eingang des Verstärkers 42.

Diese Daten sind zeitlich so angeordnet, daß sie nach dem Kennungsignal, welches wieder aus einem Impuls besteht, der eine größere Amplitude aufweist als die übrigen analogen Signalanteile, ein Zeitbereich ohne Signalübertragung erscheint, welcher ausreichend ist, um alle weiteren an Eingang des Verstärkers 42 erscheinenden Signale in zeitsequentieller Folge aufzunehmen.

Dabei werden durch die Programmierung des Systems und den Taktdecoder 47 jeweils bezüglich des Eingangs der Schaltung 42 für eine Anzahl Takte ausschließlich Signale über die Leitung vom Ausgang der Baugruppe 50 aufgenommen - entsprechend der Anzahl der auf dieser Leitung in sequentieller Folge erscheinenden Signale. Nach dem Kennungssignal werden in dem darauffolgenden Zeitfester ohne Signale vom Ausgang der Schaltung 50 die übrigen Eingänge des Verstärkers 42 nacheinander abgefragt, so daß am Aufgang der Schaltung 42 alle Eingangssignale der Schaltung 50 und alle weiteren Eingangssignale der Schaltung 42 in zeitsequentieller Folge erscheinen und nachfolgend in üblicher Weise weiterverarbeitet werden können.

Die nachfolgenden Schaltungen 43 und 44 werden also jeweils - wie für die Verarbeitung des jeweiligen analogen Eingangssignals vorgesehen - umgeschaltet, auch wenn der Eingang der Schaltung 42 für einige Taktsequenzen mit dem Ausgangs der entfernt gelegenen Schaltung 50 verbunden bleibt.

Voraussetzung für diese Betriebsweise ist eine Synchronisation des Taktgebers 51 mit der Taktgabe im Hauptsystem.

Das wird dadurch erreicht, daß der Taktdecoder in diesem Fall nicht - oder nicht ausschließlich - vom Systembus 2 angesteuert wird, sondern von einem separaten Taktgenerator 56, der seinerseits durch ein PLL-System gesteuert wird, welches vom Ausgang des Decoders 55 synchronisiert wird. Der Bezugsimpuls innerhalb der Ausgangssignale der Schaltung 50 sorgt also dafür, daß Taktgenrator 56 phasensynchron mit der von außen zugeführten sequentiellen (multiplexen) Signalfolge übereinstimmt.

Da durch das PLL-System die Phasenlage der Gesamtsequenzen übereinstimmt, lassen sich auch die einzelnen Signalanteile im Multiplex-Signal zeitlich synchron nacheinander erfassen, so daß die Einbindung der vom Ausgang der Schaltung 50 eingehenden sequentiellen Signalanteile in das von der Schaltung 42 erzeugte Zeitschema exakt erfolgen kann.

Die Rückführung von Signalen zur weiteren Verarbeitung vom Ausgang der Multiplexanordnung zurück zu deren Eingang soll am Beispiel des Signals dargestellt werden, welches der Baugruppe (Sample&Hold-Schaltung) 58 zugeführt wird. Dabei soll es sich um das der Körpertemperatur entsprechende Signal handeln, welches von der Schaltung 22 abgegeben wird. Dieses Signal steht am Ausgang der Schaltung 58 zur Verfügung. Die Übertragung dieses Signals innerhalb der Multiplex-Signalfolge erfolgt bevorzugt nur in größeren Zeitabständen, da die Änderungsrate gering ist. Auf diese Weise steht innerhalb des Kanals mehr Zeit für sich schneller ändernde oder weitere Signale zur Verfügung. Häufig ist für die Signalverarbeitung im Schrittmacher aber nicht der Absolutwert der Temperatur, sondern deren zeitliche Änderung oder auch das Integral dieser Größe erforderlich. Zur entsprechenden analogen Verarbeitung wird das Ausgangssignal der Baugruppe 58 wieder zum Eingang der Baugruppe 42 zurückgeführt und in einem anderen zeitlichgen Fenster erneut unter jeweils sequentieller Einschaltung der betreffenden Zeitkonstanten-(Filter-) glieder ind der Schaltung 44 erneut verarbeitet. Auch diese Verarbeitung kann mit einer geringen Abtastrate erfolgen. Auf diese Weise lassen sich die speziellen Anforderungen der verschiedensten analogen Eingangssignale optimal erfüllen, wobei die gesamte analoge Signalverarbeitung im wesentlichen in einem einzigen Übertragungskanal erfolgen kann.

Unter Bezugnahme auf Figur 4 soll der dort dargestellte externe, diskrete Schaltungsteil näher beschrieben werden. Er besteht aus Eingangskondensatoren C1 und C2, welche Schutz gegen elektromagnetische Einstreuungen gewähren. Je eine Dreifachanordnung von Leistungs-Zenerdioden D1 und D2 bietet Schutz im Falle von Defibrillationen. Anti-Aliasing und Bandbegrenzungsfilter mit Differential-Eingang werden durch Widerstände R1 bis R8 und Kondensatoren C1 bis C10 gebildet. Die Grenzfrequenzen des Eingangsbandes für den Differential-Eingangs-Filter für das Eingangssignal liegen bei 0,5 Hz und 100 Hz.

Die interne IC-Schaltung besteht aus einem Operationsverstärker Al mit Multiplexeingang und fester Verstärkung, einem Multiplex-Verstärker A2 mit programmierbarer Verstärkung und einem mehrpoligen Band Multiplex-Bandpaß-Filterverstärker A3, A4 mit fester Verstärkung, denen Komparatoren A5 bis A10 nachfolgen, welche Signalpegel detektieren, welche feste Schwellwerte überschreiten.

Der Verstärker Al mit Multiplexeingang ist in Schaltkondensator-Technologie (SC) aufgebaut, wobei die feste Verstärkung durch das Verhältnis der Eingangs- zur Rückkopplungskapazität bestimmt wird. Bei dem bevorzugten Ausführungsbeispiel beträgt die Eingangskapazität 25 pF und die Rückkopplungskapazität 1 pF entsprechend einer festen Verstärkung von 25.

Der Programmierschalter ermöglicht eine unipolare/bipolare Arbeitsweise. In der Weise, daß die Eingangsverstärkertypologie entweder als unsymmetrischer Verstärker (Schalter offen) oder Differential-Verstärker (Schalter geschlossen) betrieben wird. Die Steuerschaltung für die Phase Φ-F bietet Austastsignale für den Eingang und Schutz vor Stimulationsimpulsen des selben Kanals oder der entfernten Feldern von Stimulationsimpulsen des anderen Kanals, welche an der sensenden Elektrode auftreten. Die Betriebweise des Multiplex-Verstärkers mit geschalteteten Kondensatoren ist abhängig von der Phasenlage von Schaltern bildenden Kondensatoten, welche von einem Taktgenerator mit zeitlich nicht überlappenden Impulsen gesteuert wird. Das Multiplexen der beiden Eingangssignale wird durch das Schalten jeder Eingangsstufe mit einer Hälfte der Abtastrate des Rückkopplungnetzwerkes des Verstärkers mit unsymetrischem Eingang bewirkt. Die Schaltsequenz ist in Figur 7 dargestellt. Während die als Austastschalter wirkenden Kondensatoren das Eingangssignal impulsweise kurzschließen, um in Zeitsegmenten, während der durch Ausgangssignale Störungen auftreten können kurzschließen, erzeugen die schaltbaren Kondensatoren in Eingangsteil des Verstärkers Al zeitweise Verindungen zwischen den Signaleingangsleitungen des Verstärkers zu den Eingangsklemmen, wie sie dem bekannten bipolaren bzw. dem unipolaren Betrieb entsprechen.

Der Verstärker A2 mit programmierbarer Verstärkung ist in unsymmetrischer, geschalteter Kondensatortypologie mit einer Verstärkung, welche durch das Verhältnis von Eingangs- und Rückkopplungkapazität festgelegt wird. Bei dem bevorzugten Ausführungbeipiel ist die Eingangskapazität programmierbar in Stufen von 1, 2, 4 und 8 pF. Für den Fall, daß die Rückkopplungkapazität 0,6 pF beträgt, ist die Verstärkung programmierbar von 1,667 bis 25 in Stufen von 1,667. Eine maximale Über-alles-Verstärkung von 625 (25 mal 25) wird bei dem bevorzugten Ausführungsbeipiel von dem Eingangsverstärker und dem programmierbaren Verstärker gemeinsam erreicht. Die Betriebweise des Verstärkers mit geschalteten Kondensatores ist abhängig von der korrekten Phasenlage der Schalter, welche von dem Taktgenerator mit nicht überlappenden Takten gesteuert wird. Die Schaltfolge ist in Figur 7 wiedergegeben. Die geschalteten Kapazitäten wirken entsprechend den Rückkopplungs- und Eingangswiderstäden einer bekannten Operationverstärkerschaltung bei kontinuierlichem Betrieb.

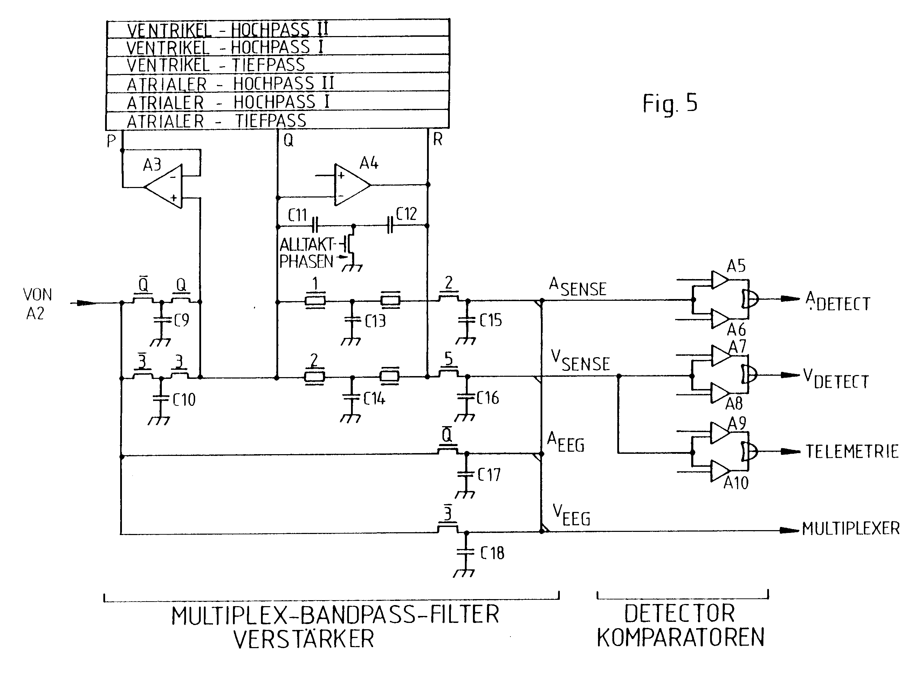

Unter Bezugnahme auf Figur 5 soll das Multiplex-Mehrpol-Bandpaßfilter mit fester Vertärkung näher beschrieben werden. Es ist in geschalteter Kondesatortechnologie hergestellt mit Verstärkungs- und Bandpaßeigenschaften, welche durch das Verhältnis der Kondensatorelemente in Rückkopplungs- und Eingangsnetzwerk festgelegt werden. Bei dem bevorzugten Ausführungsbeipiel wird das Ausgangssignal des Verstärkers mit programierbarer Verstärkung nach einem Sample&Hold-Prinzip ermittelt, wobei die breitbandigen atrialen ind ventrikulären EKG-Signale in zwei Gruppen unterteilt werden. Die eine abgetastete Gruppe wird dem Eingang der Filterschaltung zugeführt, während die andere zur Telemetrie-Überwachungs-Einheit gelangt. Die das Übertragungsverhalten bestimmenden Filterglieder entsprechen in ihrem Aufbau den bekannten Filterschaltungen für kontinuierlichen Betrieb, wobei, bedingt durch die geschalteten Kapazitäten der Stromfluß impulsweise erfolgt. Die geschalteten Kapazitäten entsprechen dabei den Widerständen. Frequenzbestimmende Kondensatoren sind in herkömmlicher Weise ausgebildet, so daß diese Kondensatoren nur geladen oder entladen werden, wenn sie über die geschalteten Kapazitäten mit anderen Schaltungspunkten verbunden sind. Diese herkömmlichen Kondensatoren speichern also ihre Ladungen bis sie in der sequentiellen Folge wieder "bedient" werden. Zwischen diesen sequentiellen Taktzeiten ist ihr Zustand unveränderlich also "eingefroren", wenn man einmal von der natürlichen Selbstentladung absieht.

Die abgetasten Breitband-EKG-Eingangssignale werden gehalten und sequentiell gefiltert durch die Filterschaltung und das mehrpolige Rückkopplungnetzwerk, um gefilterte atriale und ventrikuläre EKG-Ausgangssignale zu erhalten. Diese Ausgangssignale werden sowohl dem Telemetrieüberwachungs als auch den Detektorschaltkreisen zugeführt. Die Multiplexfilter werden in unsymmetrischer Verstärker-Topologie erzeugt, wobei zwei atriale Hochpaß- und ein atrialer Tiefpaß-Bereich vorgesehen ist, sowie zwei ventrikulärer Hochpaß- und ein ventrikulärer Tiefpaß-Bereich. Die sequentielle Phasenschaltung der Schalter wird gesteuert durch einen Taktgenerator mit nichtüberlappenden Impulsen. Die sequentiellen Phasen lagen der Taktsignale des Hochpaß- und Tiefpaß-Filters, erzeugen die gewünschten gefilterten Multiplexausgangssignale. Die Schaltimpulsfolge ist in Figur 7 dargestellt. Die sequentielle Betriebsweise der Schalter und der Hochpaß- bzw. Tiefpaß-Filterbereiche erzeugen gefilterte Ausgangssignale mit +80 db/pro Dekade im tieffrequenten Bereich und -40 db/pro Dekade im hochfrequenten Bereich.

Jede Filterschaltung trägt einen Anteil von 40 db/Dekade bei, so daß jeder Kanal zwei Hochpaß-Netzwerke und ein Tiefpaß-Netzwerk benötigt, um das gewünschte Filterverhalten zu erzeugen.

Die Eckfrequenzen werden durch die Kondensatorverhältnisse innerhalb jeder Filterschaltung festgelegt, wodurch die -3 db Eckfrequenzen zu 40 und 100 für den Vorhof, und mit 20 und 60 Hz für den Ventrikel festgelegt werden.

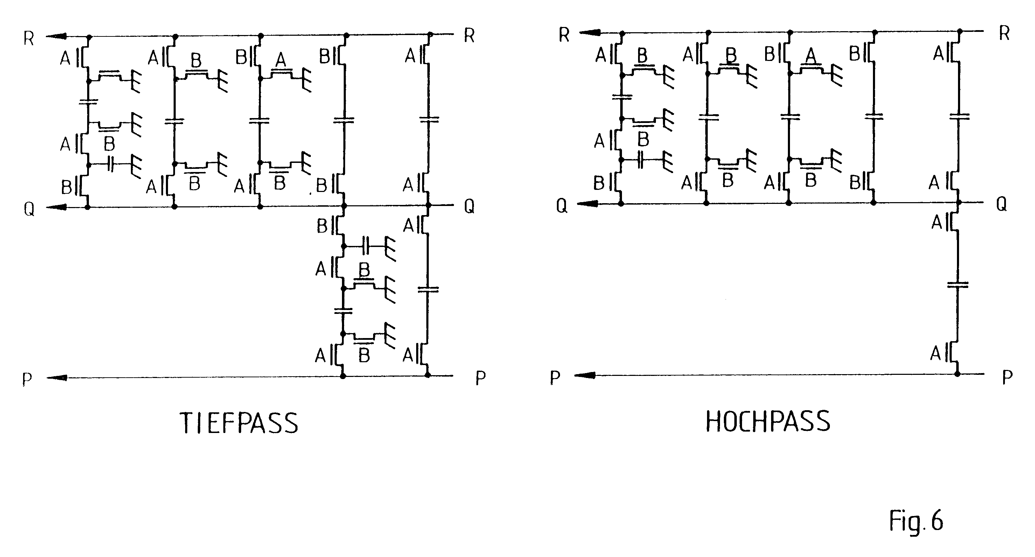

Die Rückkopplungswege der Filterschaltungen gemäß Figur 5 (obere Blöcke) sind in Figur 6 dargstellt.

Bezüglich der Verbindungswege zwischen den in den Figuren 4 bis 6 dargestellten Bauelemente wird auf die Stromlaufpläne bezuggenommen. Hinsichtlich der Einschaltzeiten der geschalteten Kapazitäten wird auf die entsprechend gekennzeichneten Zeitintervalle und die zugeordneten Bezeichnungen der betreffenden Betriebsfunktionen verwiesen. Es ist ersichtlich, daß einige der schaltbaren Kondensatoren mit jedem zweiten Taktimpuls in der Impulssequenz getaktet - also aktiviert werden -, während andere Kondensatorgruppen alle vier, acht oder zwölf Impulse aktiviert werden. Auf diese Weise lassen sich durch die sequentielle Betriebsweise nachgebildete verzweigte, Signalwege in der Nachbildung erneut verzweigen oder in drei weitere Wege aufteilen (entsprechend der Taktung mit jedem achten oder zwölften Signalimpuls. Werden also beispielsweise die vom Verntrikel und Atrium aufgenommenen Signale abwechselnd über denselben Verstärker- und Filterweg verarbeitet, so lassen sich für Atrium- und Ventrikelsignale noch jeweil unterschiedliche Verstärkungs- oder Filtercharakteristiken einstellen. Die Vielfalt dieser "Auffächerung" von Signalverarbeitungswegen wird durch die erforderliche obere Grenzfrequenz bei der Signalübertragung bestimmt, da höchsten zu übertragenden Frequenzen durch die sequentielle Übertragung beeinträchtigt werden.

Die dargestellten Schaltungen enthalten die Verbindungswege der sequentiell nacheinander aktivierbaren Schaltungskonfigurationen überlagert, wobei die taktweise geschalteten Kondensatoren die Schaltelemente in den Verbindungswegen bilden, welche periodisch nacheinander aktiviert werden. In frequenzbestimmenden Schaltungen, wie Filtern, bilden diese Kapazitäten auch gleichzeitig die frequenzbestimmenden Schaltelemente, da ihre Werte so gewählt sind, daß sie - bezogen auf die Taktzeiten - unterschiedliche dynamische Widerstände bilden.

Die Änderung der Übertragungseigenschaften des multiplexen Übertragungswegs kann auch über den Digitalteil über einen entsprechenden Schaltzustand der jeweiligen geschalteten Kapazitäten in Abhängigkeit von der Auswertung der in den jeweiligen sequentiellen "Kanälen" übertragenen Daten erfolgen.

Auf diese Weise ist eine Amplitudenregelung und auch eine Änderung der Filtereigenschaften in Abhängigkeit von den Signalamplituden auf der Leitung möglich. Die Leitung mit ihren komplexen schaltbaren Übertragungseigenschaften kann dabei auf einem oder mehrenen zeitlichen Übertragungskanälen aufgrund einer für dessen Zeitsequenz programmierten Übertragungsfunktion auch einen "Regler" oder ein sonstiges mit Zeitkonstanten oder Filtereigenschaften behaftetes Glied bilden, wie es sich allgemein mit komplexen Übertragungsfunktionen beschreiben läßt.

Die Erfindung beschränkt sich in ihrer Ausführung nicht auf das vorstehend angegebene bevorzugte Ausführungsbeispiel. Vielmehr ist eine Anzahl von Varianten denkbar, welche von der in den Ansprüchen definierten Lösung auch bei grundsätzlich anders gearteten Ausführungen Gebrauch machen.

## Patentansprüche

1. Implantierbarer Herzschrittmacher mit mehreren Eingängen zur Signalaufnahme (5, 6, 19, 20, 22, 32, 33) und mit diesen Eingängen verbundenen Schaltmitteln (11) zur Signalverarbeitung, bei dem von externen Quellen und/oder von internen Quellen über Signalaufnehmer erhaltene analoge Signale auf einem gemeinsamen Signalübertragungsweg (2) zeitlich sequentiell übertragen werden,
**dadurch gekennzeichnet,** daß
synchron zu der Umschaltung der Verbindungen von den Signalaufnehmern (5, 6, 19, 20, 22, 32, 33) zu dem Signalübertragungsweg einzelne oder mehrere der Verstärkungs- und oder Filtereigenschaften verändert werden, wobei die Umschaltung der Verbindungen und Verstärkungs- und/oder Filtereigenschaften mittels umschaltbarer Kondensatoren (C15 bis C18) erfolgt.

2. Herzschrittmacher nach Anspruch 1, **dadurch gekennzeichnet,** daß die Schaltung mit den umschaltbaren Kondensatoren (C15 bis C18) in integrierter Form aufgebaut ist.

3. Herzschrittmacher nach Anspruch 1 oder 2, **dadurch gekennzeichnet,** daß im Signalübertragungsweg vorgesehenen Filterschaltungen (44; A3, A4) die schaltbaren Kondensatoren (C15 bis C18) die Filtereigenschaften mitbestimmende Kapazitäten bilden.

4. Herzschrittmacher nach Anspruch 1, **dadurch gekennzeichnet,** daß bei im Signalübertragungsweg vorhandenen Verstärkerschaltungen (43, A2) die Verstärkung durch Umschaltung der Eingangs- bzw. Rückopplungskapazität veränderbar ist.

5. Herzschrittmacher nach einem der vorangehenden Ansprüche, **dadurch gekennzeichnet,** daß Schaltsignale zur Umschaltung der Verbindungen von den Signalaufnehmern (5, 6, 19, 20, 22, 32, 33) von einem nachgeschalteten digital arbeitenden System (8; 56, 57) erzeugt werden.

6. Herzschrittmacher nach Anspruch 5, **dadurch gekennzeichnet,** daß die Schaltsignale über einen entsprechenden Decoder (47) über den Datenbus (2) des digital arbeitenden Systems abgeleitet werden.

7. Herzschrittmacher nach einem der vorangehenden Ansprüche, **dadurch gekennzeichnet,** daß die Anschalthäufigkeit der einzelnen Signalquellen an den Übertragungsweg unterschiedlich und insbesondere veränderbar ist.

8. Herzschrittmacher nach einem der vorangehenden Ansprüche, **dadurch gekennzeichnet,** daß dem Signalübertragungsweg Analog-/Digital-Wandler (23), insbesondere Schwellwertdetektoren, nachgeschaltet sind, deren Eingänge sequentiell mit dem Signalübertragungsweg verbindbar sind.

9. Herzschrittmacher nach Anspruch 8, **dadurch gekennzeichnet,** daß eine Einrichtung vorgesehen ist, durch die bei Erreichen eines vorgegebenen Signalamplitudenwerts ein Interruptsignal an einen digitalen Prozessor (8) abgegeben wird.

10. Herzschrittmacher nach einem der vorangehenden Ansprüche, **dadurch gekennzeichnet,** daß dem Signalübertragungsweg eine Sample&Hold-Schaltung (46) nachgeschaltet ist, deren Eingang sequentiell mit dem Signalübertragungsweg verbindbar ist.

11. Herzschrittmacher nach einem der vorangehenden Ansprüche, **dadurch gekennzeichnet,** daß die Signalaufnehmer die atriale und die ventrikuläre Elektrode (5, 6) bilden.

12. Herzschrittmacher nach einem der vorangehenden Ansprüche, **dadurch gekennzeichnet,** daß die Signalaufnehmer (22, 49) solche für weitere körpereigene Signale bilden, welche unabhängig von elektrischen Signalpotentialen im Herzen aufnehmbar sind, insbesondere Temperatur-, Druck-, Schall-, plethysmographische, photometrische oder mechanische oder chemische Signale.

13. Herzschrittmacher nach einem der vorangehenden Ansprüche, **dadurch gekennzeichnet,** daß im Ventrikel neben der Stimulationselektrode (6) eine weitere Elektrode zur elektrischen Kontaktierung der Herzwand vorgesehen ist.

14. Herzschrittmacher nach einem der vorangehenden Ansprüche, **dadurch gekennzeichnet,** daß Meßwertaufnehmer als Strom- oder Spannungsaufnehmer (19, 20) im Batterie- oder Spannungsvervielfacher-Stromkreis ausgebildet ist.

15. Herzschrittmacher nach einem der vorangehenden Ansprüche, **dadurch gekennzeichnet,** daß der Ausgang des Signalübertragungswegs unter Einschluß eines Zwischenspeichers (30) mit seinem Eingang verbindbar ist, wobei die Verbindung des Zwischenspeichers mit dem Ein- bzw. Ausgang zu unterschiedlichen Zeiten erfolgt.

16. Herzschrittmacher nach Anspruch 15, **dadurch gekennzeichnet,** daß zu mindestens einer der Zeiten der Signalübertragungsweg eine komplexe Übertragungsfunktion aufweist.

17. Herzschrittmacher nach einem der vorangehenden Ansprüche, **dadurch gekennzeichnet,** daß der Signalübertragungsweg mit zeitsequentieller (multiplexer) Übertragung eine Leitung (26) zum Schrittmacher außerhalb seines Gehäuses einschließt.

18. Herzschrittmacher nach Anspruch 17, **dadurch gekennzeichnet,** daß die Umschaltrate der Signale auf der Leitung (26) außerhalb des Gehäuses geringer ist als innerhalb und bei gleicher Wiederholungsrate pro übertragenem Signal dem im Inneren des Gehäuses verlaufenden Übertragungsweg weitere Signale innerhalb des oder der verbleibenden Zeitfenster(s) zeitsequentiell zugefügt werden.

19. Herzschrittmacher nach Anspruch 17, **dadurch gekennzeichnet,** daß außerhalb des Gehäuses ein separater Taktgeber (56) zur Steuerung der wechselweisen Anschaltung der Signalquellen (5, 6, 19, 20, 22, 32, 33) an den Übertragungsweg vorgesehen ist und Bezugsimpulse für die Synchronisation eines innerhalb des Schrittmachers vorgesehenen Umschalters zwischen Übertragungsweg und Signalausgängen auf dem Übertragungsweg selbst ohne zusätzliche Signalader mitübertragen werden.

20. Herzschrittmacher nach einem der vorangehenden Ansprüche, **dadurch gekennzeichnet,** daß ein redundanter Zeitgeber (51) für Schrittmacherimpulse vorgesehen ist, der von der Eingangsseite des Signalübertragungswegs her gesteuert wird.

21. Herzschrittmacher nach einem der vorangehenden Ansprüche, **dadurch gekennzeichnet,** daß eine Einrichtung zur Umschaltung zwischen bipolarem und unipolarem Elektrodenanschluß mit schaltbaren Kondensatoren vorgesehen ist.

22. Herzschrittmacher nach einem der vorangehenden Ansprüche, **dadurch gekennzeichnet,** daß Mittel zur digitalen Regelung der Verstärkungs- oder Filtereigenschaften des Übertragungswegs, insbesondere in der Weise, daß bei Überschreiten vorbestimmter Schwellwerte durch Signale oder Signalanteile an dessen Ausgang Verstärkungsfaktoren verkleinert werden und umgekehrt, vorgesehen sind.

23. Herzschrittmacher nach einem der vorangehenden Ansprüche, **dadurch gekennzeichnet,** daß Austastzeiten bei der Signalübertragung durch die Unterdrückung von Taktsignalen für den betreffenden zeitsequentiellen Verbindungsweg innerhalb der multiplexen Verarbeitung für die Dauer der Austastzeit hervorgerufen werden.

## Claims

1. An implantable heart pacemaker with a plurality of inputs for picking up signals (5, 6, 19, 20, 22, 32, 33) and switching means (11) connected to these inputs for signal processing, wherein analog signals received from eternal sources and/or from internal sources through signal pickups are transmitted sequentially in time along a common signal transmission path (2),
characterised in that
single or multiple amplifying and/or filtering properties are changed synchronously with the transfer of the connections from the signal pickups (5, 6, 19, 20, 22, 32, 33) to the signal transmission path, the transfer of connections and amplifying and/or filtering properties being effected by means of capacitors (C15 to C18) which can be switched over.

2. A heart pacemaker according to claim 1, characterised in that the circuit with the capacitors (C15 to C18) which can be switched over is constructed in integrated form.

3. A heart pacemaker according to claim 1 or 2, characterised in that in filter circuits (44; A3, A4) provided in the signal transmission path the switchable capacitors (C15 to C18) form the capacitances which partly determine the filtering properties.

4. A heart pacemaker according to claim 1, characterised in that in amplifier circuits (43, A2) located in the signal transmission path the amplification can be changed by switching over the input and/or feedback capacitance.

5. A heart pacemaker according to any of the preceding claims, characterised in that switching signals for transferring the connections from the signal pickups (5, 6, 19, 20, 22, 32, 33) are generated by a downstream, digitally operating system (8; 56, 57).

6. A heart pacemaker according to claim 5, characterised in that the switching signals are derived through the data bus (2) of the digitally operating system, through an appropriate decoder (47).

7. A heart pacemaker according to any of the preceding claims, characterised in that the frequency with which the individual signal sources are connected to the transmission path varies and in particular can be changed.

8. A heart pacemaker according to any of the preceding claims, characterised in that analog/digital converters (23), particularly threshold value detectors, are arranged downstream of the signal transmission path and their inputs can be connected sequentially to said path.

9. A heart pacemaker according to claim 8, characterised in that an arrangement is provided, whereby an "interrupt" signal is delivered to a digital processor (8) when a predetermined signal amplitude value is reached.

10. A heart pacemaker according to any of the preceding claims, characterised in that a sample-and-hold circuit (46) is provided downstream of the signal transmission path, and its input may be connected sequentially to said path.

11. A heart pacemaker according to any of the preceding claims, characterised in that the signal pickups form the atrial and ventricular electrodes (5, 6).

12. A heart pacemaker according to any of the preceding claims, characterised in that the signal pickups (22, 49) form electrodes for other signals from the body, which may be picked up in the heart independent of electric signal potentials, particularly temperature, pressure, sound, plethysmographic, photometric or mechanical or chemical signals.

13. A heart pacemaker according to any of the preceding claims, characterised in that an additional electrode for electrically contacting the cardiac wall is provided in the ventricle beside the stimulation electrode (6).

14. A heart pacemaker according to any of the preceding claims, characterised in that a measured value pickup is in the form of a current or voltage pickup (19, 20) in the battery circuit or voltage multiplier circuit.

15. A heart pacemaker according to any of the preceding claims, characterised in that the output of the signal transmission path may be connected to its input, taking in a buffer memory (30), the buffer memory being connected to the input or output at different times.

16. A heart pacemaker according to claim 15, characterised in that the signal transmission path has a complex transmission function at at least one of the times.

17. A heart pacemaker according to any of the preceding claims, characterised in that the signal transmission path with time-sequential (multiplex) transmission includes a line (26) to the pacemaker outside its casing.

18. A heart pacemaker according to claim 17, characterised in that the rate at which signals are switched over on the line (26) outside the casing is lower than the rate inside it and, with the same repetition rate per signal transmitted, other signals are added time-sequentially to the transmission path running inside the casing, within the remaining time window or windows.

19. A heart pacemaker according to claim 17, characterised in that a separate timer (56) for controlling the alternate connection of the signal sources (5, 6, 19, 20, 22, 32, 33) to the transmission path is provided outside the casing, and reference pulses for synchronising a change-over switch provided inside the pacemaker are transmitted between the transmission path and the signal outputs along the transmission path itself without any additional signalling wires.

20. A heart pacemaker according to any of the preceding claims, characterised in that a redundant timer (51) is provided for pacemaker pulses and controlled from the input side of the signal transmission path.

21. A heart pacemaker according to any of the preceding claims, characterised in that an arrangement for switching over between bipolar and unipolar electrode connection is provided, with switchable capacitors.

22. A heart pacemaker according to any of the preceding claims, characterised in that means are provided for digital control of the amplifying or filtering properties of the transmission path, particularly in such a way that when predetermined threshold values are exceeded by signals or parts of signals at its output amplification factors are reduced and vice versa.

23. A heart pacemaker according to any of the preceding claims, characterised in that blanking times in signal transmission are produced by suppressing clock signals for the time-sequential connection path concerned, within the multiplex processing, for the duration of the blanking time.

## Revendications

1. Stimulateur cardiaque susceptible d'être implanté, avec plusieurs entrées pour la réception de signal (5, 6, 19, 20, 22, 32, 32, 33) et avec des moyens de commutation (11) liés avec ces entrées pour le traitement de signal, lors duquel, des signaux analogiques reçus de sources externes et/ou de sources internes, par l'intermédiaire de dispositifs de réception de signal, sont transmis, séquentiellement dans le temps, sur un trajet de transmission de signal (2) commun,
caractérisé en ce que, en synchronisme avec la commutation des liaisons des dispositifs de réception de signal (5, 6, 19, 20, 22, 32, 33), au trajet de transmission de signal, une seule ou plusieurs des propriétés d'amplification et/ou de filtrage sont changées, la commutation des liaisons et des propriétés d'amplification et/ou de filtrage, ayant lieu au moyen de condensateurs (C15 à C18) susceptibles d'être commutés.

2. Stimulateur cardiaque selon la revendication 1, caractérisé en ce que le circuit avec les condensateurs (C15 à C18) susceptibles d'être commutés est constitué sous une forme intégrée.

3. Stimulateur cardiaque selon la revendication 1 ou 1 la revendication 2, caractérisé en ce que, lors de circuits de filtre (44 ; A3, A4) prévus dans le trajet de transmission de signal, les condensateurs (C15 à C18) susceptibles d'être commutés forment des capacités codéterminant les propriétés de filtrage.

4. Stimulateur cardiaque selon la revendication 1, caractérisé en ce que, lors de circuits d'amplificateur (43, A2) présents dans le trajet de transmission de signal, l'amplification est changeable par commutation de la capacité d'entrée, ou bien de la capacité de rétroaction.

5. Stimulateur cardiaque selon une des précédentes revendications, caractérisé en ce que des signaux de commutation, pour la commutation des liaisons des dispositifs de réception de signal (5, 6, 19, 20, 22, 32, 33), sont engendrés par un système (8 ; 56, 57) travaillant numériquement, mis en circuit en aval.

6. Stimulateur cardiaque selon la revendication 5, caractérisé en ce que les signaux de commutation sont dérivés, par l'intermédiaire d'un décodeur (47) correspondant, par l'intermédiaire du conducteur omnibus de données (2) du système travaillant numériquement.

7. Stimulateur cardiaque selon une des précédentes revendications, caractérisé en ce que la fréquence de mise en circuit des sources de signal individuelles, au trajet de transmission, est différente et, en particulier, susceptible d'être changée.

8. Stimulateur cardiaque selon une des précédentes revendications 1, caractérisé en ce qu'au trajet de transmission de signal, des convertisseurs analogiques/numériques (23), en particulier des détecteurs de valeur de seuil, sont mis en circuit en aval, dont les entrées sont susceptibles d'être reliées séquentiellement avec le trajet de transmission de signal.

9. Stimulateur cardiaque selon la revendication 8, caractérisé en ce qu'un agencement est prévu, grâce auquel, lors d'atteintes d'une valeur d'amplitude de signal préallouée, un signal d'interruption est délivré à un processeur (8) numérique.

10. Stimulateur cardiaque selon une des précédentes revendications, caractérisé en ce qu'au trajet de transmission de signal, est mis en circuit en aval un circuit d'échantillonnage et de maintien (46), dont l'entrée est susceptible d'être reliée séquentiellement avec le trajet de transmission de signal.

11. Stimulateur cardiaque selon une des précédentes revendication, caractérisé en ce que les dispositifs de réception de signal forment l'électrode d'oreillette (5) et l'électrode de ventricule (6).

12. Stimulateur cardiaque selon une des précédentes revendications, caractérisé en ce que les dispositifs de réception de signal (22, 49) servent pour d'autres signaux propres au corps, lesquels sont susceptibles d'être reçus indépendamment de potentiels de signal électriques dans le coeur, en particulier des signaux de température, de pression, de bruit, pléthysmographiques, photométriques, mécaniques, ou chimiques.

13. Stimulateur cardiaque selon la revendication 1, caractérisé en ce que, dans le ventricule, à côté de l'électrode de stimulation (6), une autre électrode est prévue pour la mise en contact électrique de la paroi de coeur.

14. Stimulateur cardiaque selon une des précédentes revendications, caractérisé en ce que des dispositifs de réception de valeur de mesure sont constitués, comme des dispositifs de réception de courant ou de tension (19, 20), dans le circuit de courant de batterie ou le circuit multiplicateur de tension.

15. Stimulateur cardiaque selon une des précédentes revendications, caractérisé en ce que la sortie du trajet de transmission de signal, avec inclusion d'une mémoire intermédiaire (30), est susceptible d'être reliée avec son entrée, la liaison de la mémoire intermédiaire avec l'entrée ou bien la sortie ayant lieu à des temps différents.

16. Stimulateur cardiaque selon la revendication 15, caractérisé en ce qu'au moins à un des temps, le trajet de transmission de signal présente une fonction de transmission complexe.

17. Stimulateur cardiaque selon une des précédentes revendications, caractérisé en ce que le trajet de transmission de signal, avec transmission séquentielle dans le temps (multiplexeur), inclut une ligne (26) au stimulateur cardiaque, à l'extérieur de son boîtier;

18. Stimulateur cardiaque selon la revendication 17, caractérisé en ce que la vitesse de commutation des signaux est plus faible sur la ligne (26) à l'extérieur du boîtier qu'à l'intérieur, et, lors de vitesse de répétition constante par signal transmis au trajet de transmission s'étendant à l'intérieur du boîtier, d'autres signaux sont ajoutés séquentiellement à l'intérieur de la, ou des, fenêtre(s) de temps restant libre(s).

19. Stimulateur cardiaque selon la revendication 17, caractérisé en ce qu'à l'extérieur du boîtier, un générateur de cycle (56), pour la commande de façon alternée de la mise en circuit des sources de signal (5, 6, 19, 20, 22, 32, 33) au trajet de transmission, est prévu et des impulsions de référence, pour la synchronisation d'un commutateur prévu à l'intérieur du stimulateur cardiaque entre trajet de transmission et sorties de signal, sont cotransmises sur le trajet de transmission lui-même, sans conducteur de câble de signal supplémentaire.

20. Stimulateur cardiaque selon une des précédentes revendications, caractérisé en ce qu'un générateur de temps (51) redondant, pour des impulsions de stimulateur cardiaque, est prévu, qui est commandé à partir du côté d'entrée du trajet de transmission de signal.

21. Stimulateur cardiaque selon une des précédentes revendications, caractérisé en ce qu'un agencement, pour la commutation entre raccordement d'électrode bipolaire et raccordement d'électrode unipolaire, avec des condensateurs susceptibles d'être commutés, est prévu.

22. Stimulateur cardiaque selon une des précédentes revendications, caractérisé en ce que des moyens, pour la régulation numérique des propriétés d'amplification ou de filtrage du trajet de transmission, en particulier de façon telle, que lors de franchissements de valeurs de seuil prédéterminées par des signaux ou des parties de signaux à sa sortie, des facteurs d'amplification sont réduits et inversés, sont prévus.

23. Stimulateur cardiaque selon une des précédentes revendications, caractérisé en ce que des temps d'effacement, lors de la transmission de signal, sont suscités par la suppression de signaux de synchronisation pour le trajet de liaison concerné séquentiellement dans le temps, à l'intérieur du traitement multiplex, pour la durée du temps d'effacement.
